# EUROPEAN PATENT SPECIFICATION

(11) **EP 2 911 647 B1**
(45) Date of publication and mention of the grant of the patent: **07.03.2018**
(21) Application number: 13779881.5
(22) Date of filing: 23.10.2013
(51) Int. Cl.: A61K 9/00, A61K 9/16, A61K 9/70

(54) **PROCESS FOR THE MANUFACTURING OF A MULTILAYER DRUG DELIVERY CONSTRUCT**
VERFAHREN ZUR HERSTELLUNG EINES MEHRSCHICHTIGEN ARZNEIMITTELFREISETZUNGSKONSTRUKTS
PROCÉDÉ POUR LA FABRICATION D'UN PRODUIT DE RECOMBINAISON D'ADMINISTRATION DE MÉDICAMENT MULTICOUCHE

(30) Priority: 23.10.2012 EP 12189563; 23.10.2012 US 201261717205 P
(43) Date of publication of application: 02.09.2015
(73) Proprietor: DSM IP Assets B.V., 6411 TE Heerlen (NL)
(72) Inventor: ZUPANCICH, John Andrew, NL-6100 AA Echt (NL); BERARD, Julien François, NL-6100 AA Echt (NL)
(74) Representative: DSM Intellectual Property
(86) International application number: PCT/EP2013/072142
(87) International publication number: WO 2014/064140

(56) References cited:
- WO-A1-2009/012449
- WO-A1-2011/045443
- US-A1- 2007 134 332

## Description

The present invention relates to a process for the manufacturing of a multilayer drug delivery construct based on at least one drug loaded film. The present invention further relates to the multilayer drug delivery construct obtainable by the process of the present invention. The invention also relates to the use of the multilayer construct in ophthalmology, cardiovascular, pain management, musculoskeletal, cancer treatment in central nervous system (CNS) or in vaccines delivery.

The development of drug delivery constructs for controlled and/or sustained release of drugs is an area of interest in the pharmaceutical industry. Known drug delivery constructs are for example particles such as micro-and nanoparticles, fibers, rods, films or coatings. The drug delivery constructs often comprise a drug dispersed in a biocompatible polymer matrix which can be implanted, administered orally or injected. The polymers most often used are for example poly-(lactic acid) or poly- (lactic acid-co-glycolic acid).

Drug delivery constructs capable of providing a sustained, local delivery of therapeutics provide unique opportunities and more efficacious strategies for the treatment of various pathologies. The requisite therapeutic dose together with the desire for prolonged release durations often necessitates high drug loadings in constructs to provide efficacious controlled release solutions. Additionally, the sensitivity of, for example therapeutic biologics, to local environmental stresses often compels the formulation of drugs with a combination of stabilizing excipients. The required mass or volume of this combined payload (drugs + excipients), coupled with conventional (practical) constraints on drug delivery construct size, often results in a drug delivery construct with relatively high weight (and/or volume) fractions of payload relative to the overall drug delivery construct mass (and/or volume).

Drug delivery constructs with a high loading of drugs (i.e., high solid content of payload relative to polymer) typically exhibit uncontrolled and rapid release of the drugs into the surrounding medium. This is due in part to isolated drug-rich domains present at the surface of the construct that have direct access to the surrounding medium.

When the construct is placed in a medium the payload rapidly solubilizes and releases (i.e. diffuses) out of the construct. If moreover the total volumetric loading of drug-rich domains in a construct exceeds a critical value (e.g., the percolation threshold or critical porosity range, Siegel, R.A. et al., J. Controlled Release 8 (1989) 223-236), the close proximity of domains within the matrix leads to clustering and interconnectivity. If then any part of this interconnected domains has access to the medium, solubilization and diffusion of drugs can lead to a rapid release of large fractions of the total drug-load. The extent to which the percolation threshold is exceeded influences the extent of domain interconnectivity within a construct and in turn, the overall fraction of drug-load that will rapidly be released from the construct. The actual volume fraction of payload needed to reach the percolation threshold (i.e., critical volumetric loading) within a given construct is dependent on a number of factors such as the construct dimensions, particle (domain) size, particle (domain) size distribution, homogeneity of the particle distribution within the matrix, ratio of particle size to construct thickness, etc.

The object of the present invention is to overcome or mitigate the above mentioned disadvantages.

Surprisingly it has been found that the above disadvantages can be overcome by providing a process for the manufacturing of multilayer drug delivery constructs comprising at least one drug loaded film, manufactured by the following steps:
(a) dissolving the polymer in a solvent
(b) mixing the dissolved polymer with a drug
(c) laminating the mixture between at least two polymeric sheets, whereby at least one polymeric sheet is permeable to the solvent,
(d) removing the sheets to provide the drug loaded film
(e) layering the drug loaded film
(f) fusing the layered film into a multilayer construct

Surprisingly the above process provides a multilayer drug delivery construct with unique opportunities to control and extend the release of the loaded drugs.

In priority filing EP11194043.3 a process is described for the manufacturing of a drug delivery system based on a polymer comprising dispersed bioactive agent or drug via the following process steps:
(a) dissolving a polymer in an organic solvent
(b) mixing the dissolved polymer with the bioactive agent
(c) laminating the mixture between at least two sheets of polymeric material,
whereby at least one sheet is permeable to the organic solvent, to provide a single layer film. A disadvantage of a single drug loaded film approaching the percolation threshold has an interconnected open pore network. The release profile of drugs from such film shows an initial burst release and a rapid release of drugs in the open pore network due to diffusion through the network.

In the present invention it has been found that the layering of at least one drug loaded film generates a multilayer drug delivery construct with improved release profiles by reducing the likelihood of interconnected pore networks but also by improving the overall homogeneity of drug distribution in the construct. Moreover the process of the present invention provides drug-loaded films with a limited solvent exposure, no elevated temperatures and improved drug dispersion.

The process of the present invention provides opportunities for the incorporation of multiple dispersed drugs within the construct and facilitates the (initial) physical separation of different drugs within the construct. Moreover it provides an opportunity for sequential release of individual drugs by controlling the properties of the layered and fused films.

In one embodiment of the present invention, the multilayer drug delivery construct may further comprise at least a non-drug loaded polymeric film. This non-drug loaded polymeric film may be included at the periphery of the multilayer construct or it may be included within the multilayer construct. The inclusion of non-drug loaded films at the periphery of the multilayer construct will improve the release profiles by limiting the amount of drug at the surface of the construct therefore decreasing burst release. Moreover it will act as a diffusion limiting membrane.

The inclusion of non-drug loaded films within the multi-layer construct will improve the release profiles by reducing the presence of interconnected pore networks that span the entire construct. One can imagine that the inclusion of non-drug loaded films providing many options for triggered or timed release based on the material properties of the non-drug loaded polymeric film.

The non-drug loaded films can be prepared via various methodologies known in the art for example via solvent casting, compression molding, lamination, etc. The non-drug loaded film may comprise the same or a different polymer as the drug loaded films.

The polymer as referred to in step (a) or as used in the non-drug loaded film can be any biostable, bioerodable or biodegradable polymer selected from the group consisting of (a) polyester homopolymers and copolymers such as polyglycolic acid (PGA), polylactic acid (PLA) including poly-L-lactic acid, poly-D- lactic acid and poly-D,L-lactic acid, poly(beta-hydroxybutyrate), polygluconate including poly-D-gluconate, poly-L-gluconate, poly-D,L-gluconate, poly(epsilon-caprolactone), poly(delta-valerolactone), poly(p-dioxanone), poly(lactic acid-co-glycolic acid) (PLGA) which can have a range of ratios of lactic acid to glycolic acid, additionally with either the racemic or meso DL lactide or the pure L-lactide, poly(lactic acid-co-delta-valerolactone), poly(lactic acid-co-epsilon-caprolactone), poly(lactic acid-co-beta-malic acid), poly(beta- hydroxybutyrate-co-beta-hydroxyvalerate), poly(1,3-bis(p-carboxyphenoxy)propane-co- sebacic acid), and poly(sebacic acid-co-fumaric acid), among others, (b) poly(ortho ester) homopolymers and copolymers such as those synthesized by copolymerization of various diketene acetals and diols, among others, (c) polyanhydride homopolymers and copolymers such as poly(adipic anhydride), poly(suberic anhydride), poly(sebacic anhydride), poly(dodecanedioic anhydride), poly(maleic anhydride), poly(1,3-bis(p-carboxyphenoxy)methane anhydride) and poly (alpha,omega-bis(p- carboxyphenoxy)alkane anhydrides) such as poly(1,3-bis(p-carboxyphenoxy)propane anhydride) and poly(1,3-bis(p-carboxyphenoxy)hexane anhydride), (d) polycarbonate homopolymers and copolymers such as poly(trimethylene carbonate), poly(lactic acid-co-trimethylene carbonate) and poly(glycolic acid-co-trimethylene carbonate) and (e) amino-acid-based polymers including tyrosine-based polyarylates (e.g., copolymers of a diphenol and a diacid linked by ester bonds, with diphenols selected, for instance, from ethyl, butyl, hexyl, octyl and benzyl esters of desaminotyrosyl-tyrosine and diacids selected, for instance, from succinic, glutaric, adipic, suberic and sebacic acid), tyrosine-based polycarbonates (f) polyesteramides, polythioesters, polyesterurethanes, polyesterurea's, polyurethanes, polyurethane acrylate, poly(amino acids), polypeptides, polyamides, polysaccharides, polyethers, polysulfones, poly(meth)acrylates, polysiloxanes or polyolefins. Any suitable blends or copolymers of these materials can also be used.

Drug delivery constructs composed of bioerodible or biodegradable polymers typically employ drug loadings sufficiently below the percolation threshold to facilitate sustained release via mechanisms such as polymer erosion or matrix swelling.

The term biostable as used herein means a material not intended to degrade in vivo during the intended lifetime.

The terms biodegradable or bioerodable as used herein refer to material which is capable of being completely or substantially degraded or eroded when exposed to an in vivo environment or a representative in vitro environment. A polymer is capable of being degraded or eroded when it can be gradually broken-down, resorbed, absorbed and/or eliminated by, for example, hydrolysis, hydration, solubilization, enzymolysis, oxidation, metabolic processes, bulk or surface erosion, and the like within a subject. It should be appreciated that traces or residue of polymer may remain following biodegradation and bioerosion.

Preferably the polymer is a biodegradable or bioerodable polymer selected from the group consisting of the above mentioned polyesters (a) or polyesteramides comprising amino-acids. Still more preferably the polyesteramide has a structure according to Formula (I), 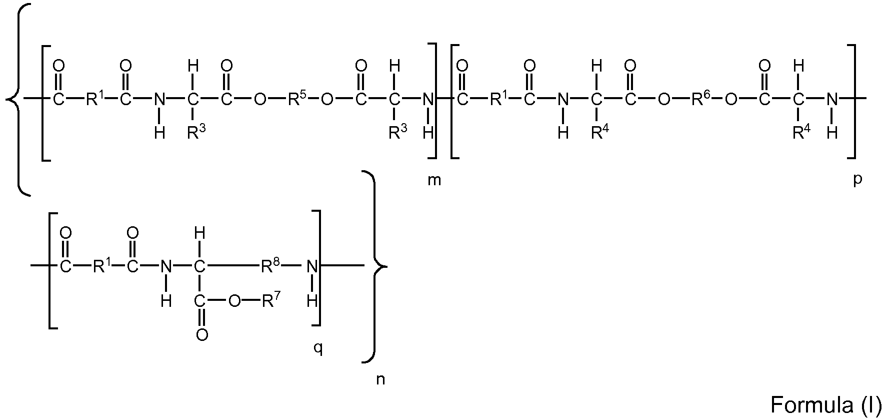 wherein
- m varies from 0.01 to 0.99; p varies from 0 to 0.99; and q varies from 0.99 to 0.01; and wherein n varies from 5 to 100; whereby
- R₁ is independently selected from the group consisting of (C₂-C₂₀) alkylene, (C₂-C₂₀) alkenylene and combinations thereof;
- R₃ and R₄ in a single backbone unit m or p, respectively, are independently selected from the group consisting of hydrogen, (C₁-C₆)alkyl, (C₂-C₆)alkenyl, (C₂-C₆)alkynyl, (C₆-C₁₀)aryl, (C₁-C₆)alkyl, -(CH₂)SH, -(CH₂)₂S(CH ₃), -CH₂OH, -CH(OH)CH₃, -(CH₂)₄NH₃+, -(CH₂)₃NHC(=NH₂+)NH₂, -CH₂COOH, - (CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂-.
- R₅ is selected from the group consisting of (C₂-C₂₀)alkylene, (C₂-C₂₀)alkenylene, alkyloxy or oligoethyleneglycol
- R₆ is selected from bicyclic-fragments of 1,4:3,6-dianhydrohexitols of structural formula (II); 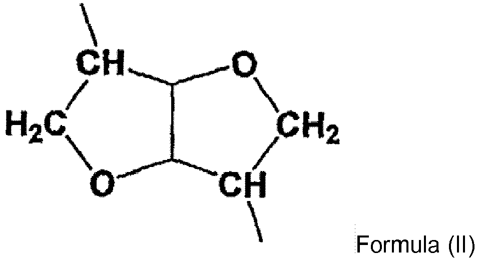
- R₇ is hydrogen, (C₆-C₁₀) aryl, (C₁-C₆) alkyl or a protecting group such as benzyl- or a bioactive agent;
- R₈ is -(CH₂)₄-;

Preferably the m, p and q units in the polyesteramide backbone of formula (I) are in a random distribution.

The biodegradable polyesteramides of Formula (I) are known in the art and for example disclosed in WO2007035938. The PEA's of Formula (I) comprise at least two linear saturated or unsaturated aliphatic diol residues into two bis-(a amino acid)-based diol-diesters. The lysine units present in backbone unit q are either protected, in case R7 is benzyl, or unprotected, in case that R7 is H. A PEA comprising unprotected lysine is further indicated as PEA-III-H, a PEA comprising protected lysine further indicated as PEA-III-Bz.

In Formula (I) m may vary from 0.01 to 0.99; p may vary from 0.2 to 3 and q may vary from 0.10 to 1.00 whereby n varies from 5 to 100; R1 is -(CH2)8; R3 and R4 in the backbone units m and p is leucine,-R5 is hexane, and R6 is a bicyclic-fragments of 1,4:3,6-dianhydrohexitols of structural formula (II); R7 may be chosen from H or a benzyl group and R8 is -(CH2)4-.

Another class of preferred polyesteramides has a structure according to Formula (III) 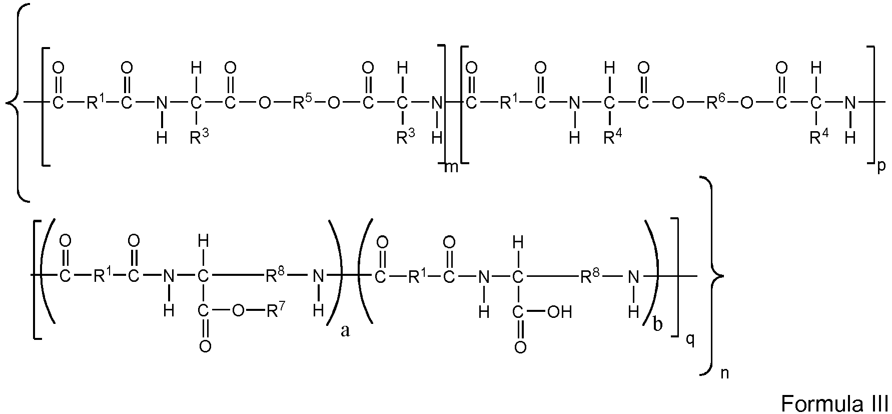 wherein
- m+p may vary from 0.9-0.1 and q may vary from 0.1 to 0.9 whereby m+p+q=1
- m or p can be 0; a is at least 0.05, b is at least 0.05 whereby a+b=1
- n may vary from 5 to 300;
- R₁ is independently selected from the group consisting of (C₂-C₂₀) alkylene, (C₂-C₂₀) alkenylene and combinations thereof;
- R₃ and R₄ in a single backbone unit m or p, respectively, are independently selected from the group consisting of hydrogen, (C₁-C₆)alkyl, (C₂-C₆)alkenyl, (C₂-C₆)alkynyl, (C₆-C₁₀)aryl, (C₁-C₆)alkyl, -(CH₂)SH, -(CH₂)₂S(CH ₃), -CH₂OH, -CH(OH)CH₃, -(CH₂)₄NH₃+, -(CH₂)₃NHC(=NH₂+)NH₂, -CH₂COOH, - (CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂-.
- R₅ is selected from the group consisting of (C₂-C₂₀)alkylene, (C₂-C₂₀)alkenylene, alkyloxy or oligoethyleneglycol
- R₆ is selected from bicyclic-fragments of 1,4:3,6-dianhydrohexitols of structural Formula II.
- R₇ is selected from the group consisting of (C₆-C₁₀) aryl (C₁-C₆) alkyl
- R₈ is -(CH₂)₄-;

Preferably the m, p and q units in the polyesteramide backbone of formula (III) are in a random distribution.

In one embodiment the biodegradable polyesteramide copolymer according to Formula (III) comprises p=0 and m+q=1 whereby m=0.75, a=0.5 and a+b=1 R₁ is (CH₂)₈, R₃ is (CH₃)₂-CH-CH₂-, R₅ is hexyl, R₇ is benzyl and R₈ is -(CH2)4-.

In another embodiment of the present invention the biodegradable polyesteramide copolymer according to Formula (III) comprises m+p+q=1, q=0.25, p=0.45 and m=0.3 whereby a is 0.5 and a+b=1 and whereby

R₁ -(CH₂)₄; R₃ and R₄ respectively are (CH₃)₂-CH-CH₂-, R₅ is selected from the group consisting of (C₂-C₂₀)alkylene, R₆ is selected from bicyclic-fragments of 1,4:3,6-dianhydrohexitols of structural formula (II), R₇ is benzyl and R₈ is -(CH2)4-.

In a still further embodiment of the present invention the biodegradable polyesteramide copolymer according to Formula (III) comprises m=0, p+q=1 and p=0.75 whereby a= 0.5 and a+b=1, R₁ is-(CH₂)₄; R₄ is (CH₃)₂-CH-CH₂-, R₇ is benzyl, R₈ is -(CH₂)₄- and R₆ is selected from bicyclic fragments of 1,4:3,6-dianhydrohexitols of structural formula (II).

In another preferred embodiment of the present invention the biodegradable poly(esteramide) copolymer according to Formula (III) comprises m+p+q=1, q=0.1, p=0.30 and m=0.6 whereby a=0.5 and a+b=1. R₁ -(CH₂)₄; R₃ and R₄ respectively, are (CH₃)₂-CH-CH₂-; R₅ is selected from the group consisting of (C₂-C₂₀)alkylene, R₇ is benzyl, R₈ is -(CH₂)₄- and R₆ is selected from bicyclic-fragments of 1,4:3,6-dianhydrohexitols of structural formula (II).

At least one of the alpha-amino acids used in the polyesteramides of Formula's (I) or (III) is a natural alpha-amino acid. For example, when the R3s or R4s are CH2Ph, the natural alpha-amino acid used in synthesis is L-phenylalanine. In alternatives wherein the R3s or R4s are -CH2-CH(CH3)2, the co-polymer contains the natural amino acid leucine. By independently varying the R3s and R4s within variations of the two co-monomers as described herein, other natural alpha -amino acids can also be used, e.g., glycine (when the R3s or R4s are H), alanine (when the R3s or R4s are CH3), valine (when the R3s or R4s are CH(CH3)2), isoleucine (when the R3s or R4s are CH(CH3)--CH2--CH3), phenylalanine (when the R3s or R4s are CH2--C6H5), lysine (when the R3s or R4s (CH2)4--NH2); or methionine (when the R3s or R4s are-(CH2)2S(CH3), and mixtures thereof.

The polyesteramide's of Formula (I) or (III) preferably have an average number molecular weight (Mn) ranging from 15,000 to 200,000 Daltons. The polyesteramide can be fabricated in a variety of molecular weights and a variety of relative proportions of the m, p, and q units in the backbone. The appropriate molecular weight for a particular use is readily determined by one skilled in the art. A suitable Mn will be in the order of about 15,000 to about 100,000 Daltons, for example from about 30,000 to about 80,000 or from about 35,000 to about 75,000. Mn is measured via GPC in THF with polystyrene as a standard.

The basic polymerization process of polyesteramides is based on the process described by G. Tsitlanadze, et al. J. Biomater. Sci. Polym. Edn. (2004) 15:1-24, however different building blocks and activating groups were used.

The polyesteramides of Formula (III) may for example be synthesized as shown in scheme 1; via solution polycondensation of para-toluene sulfonate diamines salts (X1, X2, X3, X4) with activated di-acids (Y1). Typically dimethylsulfoxide or dimethylformamide are used as solvent. Generally as a base 1.1 eq triethylamide is added with respect to the amount of para-toluene sulfonate, the reaction is carried out under an inert atmosphere at 60°C for 24-72 hours under constant stirring. Subsequently the obtained reaction mixture is purified via a water precipitation followed by an organic precipitation and filtration. Drying under reduced pressure yields the polyesteramide.

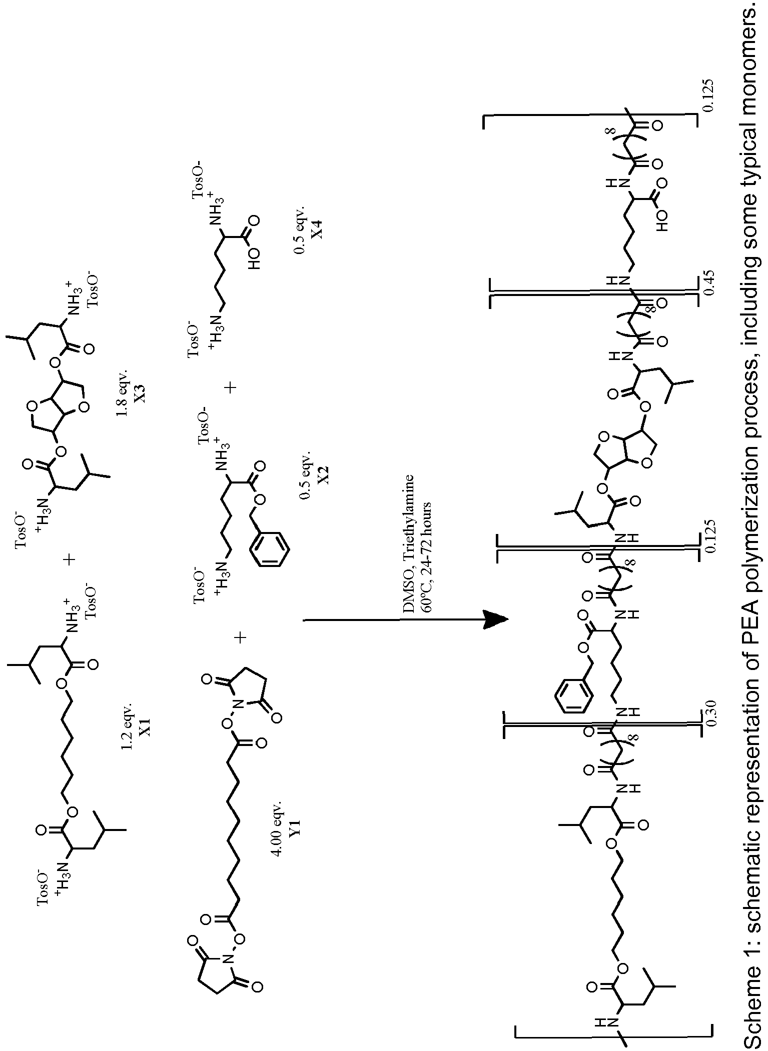

The drugs can be any agent which is a therapeutic, prophylactic, or diagnostic agent. Such drugs may include without any limitation small molecule drugs or biologics. Examples of biologics are proteins such as immunoglobulin-like proteins, antibodies, cytokines (e.g., lymphokines, monokines, chemokines), interleukins, interferons, erythropoietin (also referred to herein as "EPO"), nucleases, tumor necrosis factor, colony stimulating factors, insulin, enzymes, tumor suppressors, hormones (e.g., growth hormone and adrenocorticotrophic hormone), antigens (e.g., bacterial and viral antigens), growth factors, polypeptides and polynucleotides, such as antisense molecules. Examples of small molecule drugs can have antiproliferative or anti-inflammatory properties or can have other properties such as antineoplastic, antiplatelet, anti-coagulant, anti-fibrin, antithrombotic, antimitotic, antibiotic, antiallergic, or antioxidant properties. Examples of antiproliferative agents include rapamycin and its functional or structural derivatives, 40-O-(2-hydroxy)ethyl-rapamycin (everolimus), and its functional or structural derivatives, paclitaxel and its functional and structural derivatives. Examples of rapamycin derivatives include ABT-578, 40-0-(3-hydroxy)propyl-rapamycin, 40-O-[2-(2-hydroxy)ethoxy]ethyl-rapamycin, and 40-0-tetrazole-rapamycin. Examples of paclitaxel derivatives include docetaxel. Examples of antineoplastics and/or antimitotics include methotrexate, azathioprine, vincristine, vinblastine, fluorouracil, doxorubicin hydrochloride (e.g. Adriamycin(R) from Pharmacia AND Upjohn, Peapack NJ.), and mitomycin (e.g. Mutamycin(R) from Bristol-Myers Squibb Co., Stamford, Conn.). Examples of such antiplatelets, anticoagulants, antifibrin, and antithrombins include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein Hb/nia platelet membrane receptor antagonist antibody, recombinant hirudin, thrombin inhibitors such as Angiomax (Biogen, Inc., Cambridge, Mass.), calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor(R) from Merck AND Co., Inc., Whitehouse Station, NJ), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), super oxide dismutases, super oxide dismutase mimetic, 4-amino-2,2,6,6-tetramethylpiperidine-l-oxyl (4-amino-TEMPO), estradiol, anticancer agents, dietary supplements such as various vitamins, and a combination thereof. Examples of anti-inflammatory agents including steroidal and nonsteroidal anti-inflammatory agents include biolimus, tacrolimus, dexamethasone, clobetasol, corticosteroids or combinations thereof. Examples of such cytostatic substances include angiopeptin, angiotensin converting enzyme inhibitors such as captopril (e.g. Capoten(R) and Capozide(R) from Bristol-Myers Squibb Co., Stamford, Conn.), cilazapril or lisinopril (e.g. Prinivil(R) and Prinzide(R) from Merck AND Co., Inc., Whitehouse Station, NJ). An example of an antiallergic agent is permirolast potassium. Other therapeutic drugs which may be appropriate include alpha-interferon, pimecrolimus, imatinib mesylate, midostaurin, and genetically engineered epithelial cells. The foregoing substances can also be used in the form of prodrugs or co-drugs thereof. The foregoing substances also include metabolites thereof and/or prodrugs of the metabolites. The foregoing substances are listed by way of example and are not meant to be limiting.

The solvent as referred to in step (a) may be selected from an organic solvent for example methylene chloride, acetone, ethyl acetate, methyl acetate, tetrahydrofuran, ethanol, methanol, isopropanol or chloroform. The solvent to be used depends on the polymer to be dissolved and on the nature of the polymer sheet used in process step (c) because the solvent should be permeable to the polymer sheet. The choice can however be readily determined by one of skill in the art. In the present invention methylene chloride, chloroform, ethanol or methanol are preferably used as the solvent.

In step (b) of the process of the present invention the dissolved polymer is mixed with a drug by agitation, for example by shaking, stirring, vortexing, homogenizing or sonicating. The drug can be present, for example, as a powder, which can be crystalline, semi-crystalline or amorphous. The drug can also be present in a dispersed, dissolved or solubilized state. If present as a powder the drug is suspended in the polymer solution, if present in the liquid form the drug is dispersed in the polymer.

The weight of drug relative to the weight of dissolved polymer may range from 0.02-100 wt%. Preferably it may range from 1-75 wt%, more preferably it may range from 1-25 wt%, most preferably it may range from 5-50 wt%.

The polymer /drug mixture of step (b) in the process of the present invention may further comprise one or more excipients, including saccharides and their derivatives (e.g., disaccharides, polysaccharides, sugar alcohols), proteins (e.g., gelatin), synthetic polymers (e.g., polyvinylpyrrolidone, polyethylene glycol), antioxidants (e.g., vitamin A, vitamin E, vitamin C, retinyl palmitate), amino acids (i.e., glycine, cysteine, histidine, methionine), acids (e.g., citric acid), salts (e.g., sodium chloride), including bases, surfactants, stabilizers and/or a release modifying agent. The polymer /drug mixture may also further comprise one or more additional drugs.

In process step (c) the resulting mixture of process step (b) is laminated between at least two sheets of polymeric material, whereby at least one of the sheets is permeable to the solvent.

The sheets of solvent permeable polymeric material for example comprise a polymeric elastomer material selected from the group consisting of silicon rubber, ethylene-propylene elastomers, polyurethanes or crosslinked polymers thereof. Preferably the sheets are made from silicone rubber selected from the group consisting of a medical grade and/or food contact grade silicone rubber.

After the laminating step (c) the sheets are removed to provide a drug loaded film (d). The multilayer construct may be generated via layering (e) of the drug loaded film by for example folding or rolling. It is however also possible that more than one drug loaded film is layered. The drug loaded films may than each comprise the same or a different polymer and/or the same or a different drug. Also possible is to process the drug loaded film (d) into pieces for example by cutting which pieces are fused together to prepare at least two layers.

The multilayer construct preferably comprises at least 3 layers more preferably at least 6 layers, still more preferably at least 8 layers.

The overall thickness of the multilayer construct is controlled during or after the fusing step (f) and may range from 500 nm - 2 cm, preferably from 1 micrometer to 1 cm, more preferably from 10 micrometer to 500 micrometer, still more preferably from 50 micrometer to 250 micrometer. It is of course possible to stretch the multilayer contruct into a desired thickness.

After step (f) the process preferably comprises a shaping step in which the multilayer construct is cut and/or molded into the desired drug delivery system form for example a fiber, rod, disc, coating, particle, tube or rolled film.

The process of the present invention provides the advantage that only mild process steps are carried out with respect to temperature, pressure and presence of solvents. The process can be carried out at temperatures between 0-40 degrees Celcius, preferably between 15-30 degrees Celcius. More preferably the process of the present invention is carried out at room temperature. Room temperature means a temperature between 20-25 degrees Celsius, preferably 20 degrees Celsius.

It is known that the manufacturing of a drug delivery construct in which the drug is for example a protein causes difficult issues to deal with. Effective processes for the manufacturing of drug delivery constructs comprising the incorporation of thermally sensitive or solvent sensitive drugs, such as many proteins, peptides and polynucleotides are limited due to the typical processing conditions employed, which are often at elevated temperatures (greater than about 45 °C) and/or generate aqueous/organic interfaces (i.,e.emulsions). These process conditions may result in a significant loss of the activity of the protein.

A great advantage of the process according to the invention is that it produces a substantial homogeneous dispersion of the drug throughout the multilayer construct without using heat extrusion. The process further allows the use of process conditions such as a low temperature, limited durations of solvent exposure and a mild solvent removal. Therefore this process is in particular suitable for thermally or solvent sensitive or "labile" drugs such as many proteins, polypeptides or polynucleotides.

The term "labile" drug as used herein refers to a drug in which chemical or structural changes occur upon exposure to even benign environmental stresses (e.g., when either warmed to elevated temperatures, such as temperatures greater than physiological temperatures (about 37 °C), or dissolved in an organic solvent or in solution at an aqueous/organic interface) which can result in a substantial loss of (bio) activity and/or the development of unintended or undesired physiological responses. The process of the present invention thus allows the formation of drug delivery constructs maintaining a high degree of drug integrity, for example, protein activity present prior to processing.

The present invention further relates to a multilayer construct comprising at least two drug loaded films fused together and optional at least a non-drug loaded film layered onto or into the fused films whereby the films comprise a bioerodable polymer and whereby the drug loaded films comprise at least a dispersed drug.

The present invention further relates to a multilayer construct obtainable by the process of the present invention.

The multilayer construct is preferably further processed into a subsequently implantable or injectable drug delivery system such as a fiber, rod, disc, coating, tube or rolled film. The construct is therefore shaped via cutting and/or molding into the desired form. The construct preferably has a thickness in the range of 1 micrometer to 1 mm.

The present invention also relates to the use of the multilayer construct in ophthalmology, cardiovascular, pain management, musculoskeletal, cancer treatment, in the central nervous system or in vaccine delivery.

The present invention moreover relates to a process wherein the multilayer construct or the implantable or injectable drug delivery system is used for the release of drugs in ophthalmology, cardiovascular, pain management, musculoskeletal, cancer treatment, in central nerve system or in vaccine delivery.

The invention will now be further and specifically described by the following examples.

### FIGURES

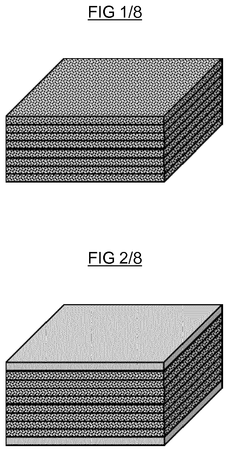

- FIG. 1:: Multi-layer construct as described in Examples 1 and 3.
- FIG. 2:: Multi-layer construct as described in Examples 2 and 4.
- FIG. 3:: Example aqueous SEC-HPLC chromatogram A) Release sample containing rHGH; B) Release medium, rHGH elutes at approximately 17 min.
- FIG.4:: Cumulative release of rhGH from multi-layer polymer devices expressed in percentage of theoretical rHGH-load as measured by aqueous SEC-HPLC in release samples of Examples 1 and 2.
- FIG. 5:: Cumulative release of rhGH from multi-layer polymer devices expressed in percentage of theoretical rHGH load as measured by aqueous SEC-HPLC in release samples of Examples 3 and 4.
- FIG. 6:: Bioactivity assay results; concentration of active rHGH in the cell culture medium as determined by the cell assay. "A" denotes release samples that have been autoclaved prior to introduction to the cell culture medium (negative control). Samples denoted with an asterisk (*): calculated concentrations of "active" rHGH exceed 500 pg/mL.

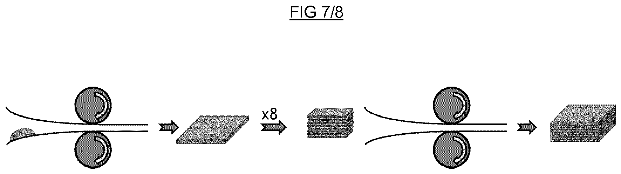

- FIG. 7:: Schematic representation of the lamination-based solvent extraction method used to generate multi-layer PEA construct containing 8 layers. The first step consists in preparing API loaded films by lamination process, the second step consisting in fusing the different layers together.

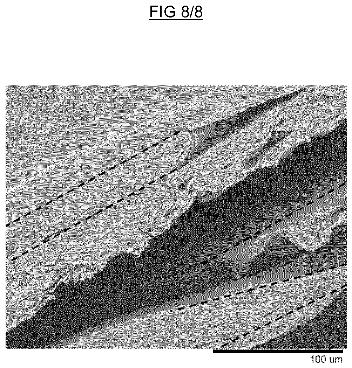

- Fig. 8:: SEM transection of a multilayer construct (N=6). The separation in between layers is highlighted by dot-lines. The two external layers are non-loaded polymer films while the 4 central layers are API-loaded.

### EXAMPLES

Necessary materials:
- Polymer solution 1: 6 wt% solution of PEA-III-Ac-Bz in dichloromethane
- Polymer solution 2: 20wt% solution of PEA-III-Ac-Bz in Methanol
- Polymer solution 3: 6 wt% solution of PEA-III-50%H in chloroform
- Polymer solution 4: 20wt% solution of PEA-III-50%H in Methanol
- Solid formulation of API: lyophilized powder of recombinant human growth hormone (rhGH; Genotropin Miniquick 2mg; Pfizer; Kent, NJ U.S.A)
- PDMS sheets: food contact grade , smooth, 1 mm thickness, approx. 10x15 cm
- Teflon sheet: PTFE coating, plain weave, 70um thickness.

### Analytical methods

The concentration of rHGH present in release samples was determined through use of aqueous size exclusion - high performance liquid chromatography (SEC-HPLC).

The method used is described below.
- Analysis of release samples were carried out on an Agilent 1200 Series system equipped with a TSKgel G2000SWXL 7.8*300 mm (TOSOH Bioscience) column, Col No 2SWX02SS4835.
- Mobile phase: 1.059 mM KH₂PO₄, 2.966 mM Na₂HPO4, 300 mM NaCl, pH=7.4, 10% EtOH (287.16 mg KH₂PO₄, 841.1 mg Na₂HPO₄, 35.64 g NaCl in 2L Milli-Q water, pH adjusted at 7.4 with NaOH 1N, 222 mL EtOH)
- Conditions: Flow 0.5 mL/min for 35 minutes, detection at 220, 250 and 280 nm.
- Response factor was calculated from a reference rHGH sample (Genotropin Miniquick 2mg; Pfizer; Kent, NJ U.S.A). Response factor was used to calculate the concentration of rHGH in release samples.
Bioactivity of rHGH present in release samples was assessed by measuring its influence on the proliferation of Nb2 (rat lymphoma) cells.¹
The method used is described below:
- Nb2 cells (Sigma-Aldrich) derived from rat T lymphoma cells were cultured in suspension in Fischer's medium supplemented with 10% fetal bovine serum, 10% horse serum, 50 µM 2-mercaptoethanol and 2% penicillin/streptomycin ("culture medium") in a humidified incubator at 37°C (5% CO₂). For proliferation assays, cells growing at log- phase were washed two times with the same medium prepared without fetal bovine serum ("incubation medium") and kept for 24 hours in this medium.
- Cells were then counted with a Guava Easycyte (Millipore) capillary cytometer using Viacount reagent (Millipore) to stain cells, according to the recommendations of the manufacturer. The cells suspension was diluted in incubation medium to reach 200.000 viable cells per mL. Cells were plated in 96-well plates (100 µL cell suspension per well).
- Samples originating from the release experiments were diluted in incubation medium to reach an expected (according to HPLC quantification) concentration of hGH between 80 and 280 pg/mL (concentration range in which growth of Nb2 cells is hGH concentration-dependent).
- These solutions were split in two aliquots and one aliquot was autoclaved. 100 µL of these solutions were added to the Nb2 cells, and cells were incubated for 72 hours at 37°C.
- After incubation, cells were stained with 50uL Viacount reagent and viable cells were counted by capillary cytometry.

### Example 1: Multi-layered construct: protein-loaded films based on PEA III Bz (n=8)

rhGH containing powder (3.7 mg solid containing 2 mg or rhGH) was dosed onto a Teflon sheet, followed by the addition of the polymer solution 1 (∼250 mg). The two components were blended by hand with a spatula until a white, opaque paste was obtained. A PDMS sheet was placed on top of the blended material, the Teflon sheet removed, and a second PDMS sheet was placed on the first. This stack was then pressed by multiple passages through a hand operated roll press. The film was allowed to sit at room temperature for five minutes after which the PDMS sheets were easily peeled away leaving a solid protein-loaded polymer film. This film was then folded tree times and pressed between PDMS sheets through uses of a hand operated roll press. The face of each PDMS sheet was briefly exposed to dichloromethane prior to pressing of the stack. After sitting for two hours at room temperature, the PDMS sheets were easily peeled away leaving a solid multi-layered protein-loaded construct. High vacuum was then applied to the construct at room temperature to remove residual solvent.

### Example 2: Multi-layered construct: protein-loaded and non-loaded films PEA-III-Bz (n=10)

Non-loaded, pure polymer thin films were obtained by solvent casting onto a polypropylene sheet. Polymer solution 2 (∼1000 mg) was applied to the substrate and a thin film formed by use of a doctor blade (gap setting ∼150µm). The film as allowed to sit at room temperature for two hours in order to facilitate solvent removal via evaporation. The resulting non-loaded polymer film had a thickness of approximately 15µm.

rhGH containing powder (3.7 mg solid containing 2 mg of rhGH) was dosed onto a Teflon sheet, followed by the addition of the polymer solution 1 (∼250 mg). The two components were blended by hand with a spatula until a white, opaque paste was obtained. A PDMS sheet was placed on top of the blended material, the Teflon sheet removed, and a second PDMS sheet was placed on the first. This stack was then pressed by multiple passages through a hand operated roll press. The film was allowed to sit at room temperature for five minutes, after which the PDMS sheets were easily peeled away leaving a solid protein-loaded polymer film. The API-loaded film was then folded tree times and sandwiched between non-loaded polymer thin films that had been prepared previously. This stack was then pressed between PDMS sheets through use of a hand operated roll press. The face of each PDMS sheet was briefly exposed to dichloromethane prior to pressing of the stack. After sitting for two hours at room temperature, the PDMS sheets were easily peeled away leaving a solid multi-layered protein-loaded construct. High vacuum was then applied to the construct at room temperature to remove all residual solvent.

### Example 3: Multi-layered construct: protein-loaded films based on PEA-X (n=8)

rhGH containing powder (3.7 mg solid containing 2 mg of rhGH) was dosed onto a Teflon sheet, followed by the addition of the polymer solution 3 (∼250 mg). The two components were blended by hand with a spatula until a white, opaque paste was obtained. A PDMS sheet was placed on top of the blended material, the Teflon sheet removed, and a second PDMS sheet was placed on the first. This stack was then pressed by multiple passages through a hand operated roll press. The film was allowed to sit at room temperature for five minutes after which the PDMS sheets were easily peeled away leaving a solid protein-loaded polymer film. This film was then folded tree times and pressed between PDMS sheets through uses of a hand operated roll press. The face of each PDMS sheet was briefly exposed to dichloromethane prior to pressing of the stack. After sitting for two hours at room temperature, the PDMS sheets were easily peeled away leaving a solid multi-layered protein-loaded construct. High vacuum was then applied to the construct at room temperature to remove residual solvent.

### Example 4: Multi-layered construct: protein-loaded and non-loaded films PEA-X (n=10)

Non-loaded, pure polymer thin films were obtained by solvent casting onto a polypropylene sheet. Polymer solution 4 (∼1000 mg) was applied to the substrate and a thin film formed by use of a doctor blade (gap setting ∼150µm). The film as allowed to sit at room temperature for two hours in order to facilitate solvent removal via evaporation. The resulting non-loaded polymer film had a thickness of approximately 15µm.

rhGH containing powder (3.7 mg solid containing 2 mg of rhGH) was dosed onto a Teflon sheet, followed by the addition of the polymer solution 3 (∼250 mg). The two components were blended by hand with a spatula until a white, opaque paste was obtained. A PDMS sheet was placed on top of the blended material, the Teflon sheet removed, and a second PDMS sheet was placed on the first. This stack was then pressed by multiple passages through a hand operated roll press. The film was allowed to sit at room temperature for five minutes, after which the PDMS sheets were easily peeled away leaving a solid protein-loaded polymer film. The API-loaded film was then folded tree times and sandwiched between non-loaded polymer thin films that had been prepared previously. This stack was then pressed between PDMS sheets through use of a hand operated roll press. The face of each PDMS sheet was briefly exposed to dichloromethane prior to pressing of the stack. After sitting for two hours at room temperature, the PDMS sheets were easily peeled away leaving a solid multi-layered protein-loaded construct. High vacuum was then applied to the construct at room temperature to remove residual solvent.

### Release experimental details

Polymer devices containing rhGH (Genotropin Miniquick 2mg; Pfizer; Kent, NJ U.S.A) were exposed to predetermined volumes of release medium at 37 °C over set periods of time. The release medium was composed of phosphate buffered saline (pH 7.4) with added Tween 20 (0.3 wt%) and sodium azide (0.1wt%). After a set period time, during which polymer devices were submerged in the release medium and incubated at 37 °C, the total volume of release medium was removed with a mechanical pipette and stored at 4 °C for subsequent analysis (i.e., referred to as release samples). A known volume of fresh release medium was then added to the polymer devices with a mechanical pipette and incubation at 37 °C continued. After set periods of time, the release medium was removed and replaced in the same manner as stated above.

### Results

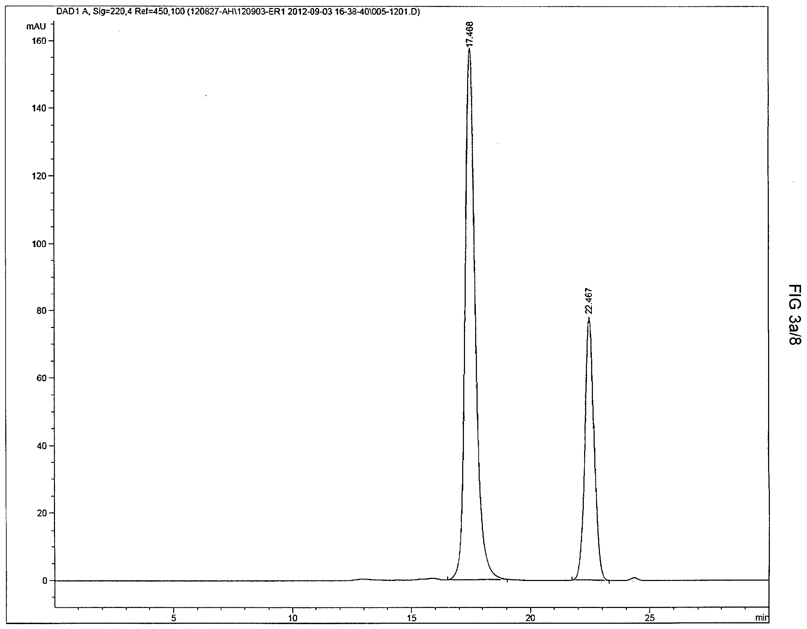

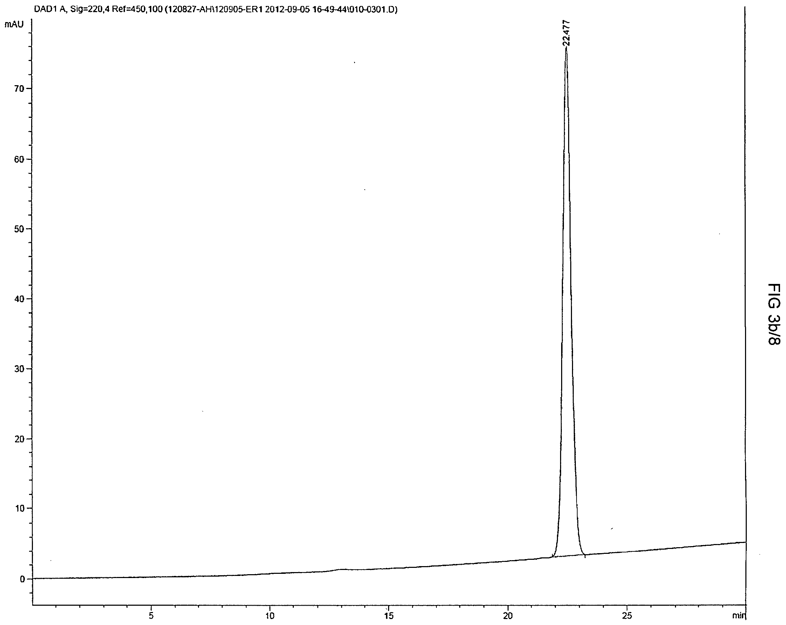

The concentration of rHGH present in release samples at time points from 1 hour to 395 hours was measured using aqueous SEC-HPLC by correlation of peak area (i.e., 17 min elution volume) to rhGH concentration through use of a calibration curve (Figure 3).

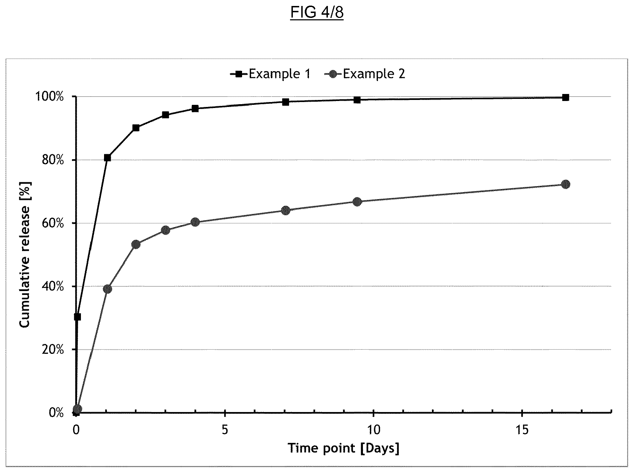

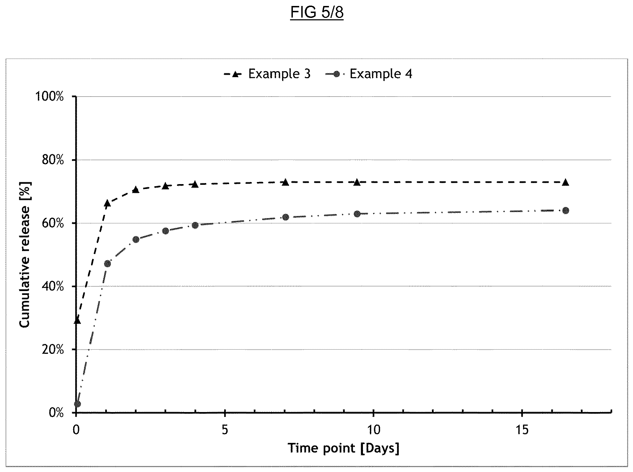

The concentration of rHGH present in release samples was expressed in cumulative percentage of protein release compared to the theoretical rhGH-load in the respective polymer devices (Figure 4 for examples 1 and 2; Figure 5 for examples 3 and 4). The presence of non-loaded polymer layers on top and bottom of devices (examples 2 and 4) show to reduce significantly the release of protein compared to non-covered devices (examples 1 and 3).

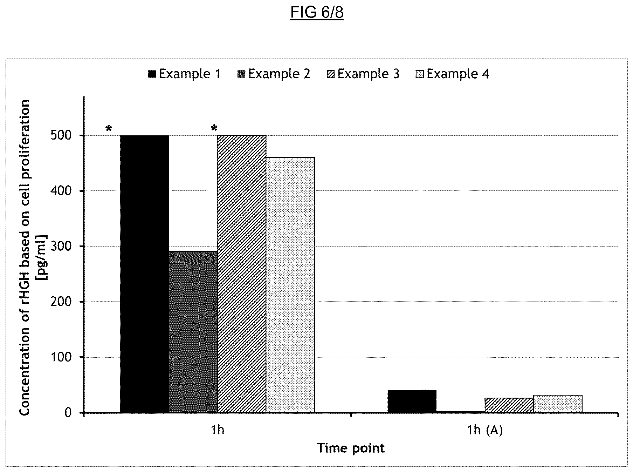

Bioactivity of rHGH released from polymer devices was measured through use of the cell-proliferation assay described above. Release samples were all diluted with and same factor and introduced to the cell culture medium. The effect of rHGH on cell proliferation was measured via cell counting with capillary cytometry. A positive cell response was measured from release samples taken at 1h indicating that rHGH released from the polymer devices was bioactive (Figure 6). As a negative control, release samples taken at 1h, were exposed to elevated temperature and pressure (i.e.., autoclave) in order to denature and/or deactivate the rHGH. A qualitative difference in cell response to release samples before and after autoclaving was recorded, validating our experimental method.

These results confirm that rHGH present in and subsequently released from the polymer devices is bioactive. These results also confirm that multi-layer constructs can be prepared that demonstrate improved release characteristics such as reduced burst release and prolonged release duration of loaded APIs.

## Claims

1. Process for the manufacturing of a multilayer drug delivery construct comprising at least one drug loaded film manufactured by the following steps:
(a) dissolving the polymer in a solvent,
(b) mixing the dissolved polymer with a drug,
(c) laminating the mixture between at least two polymeric sheets, whereby at least one polymeric sheet is permeable to the solvent,
(d) removing the sheets to provide the drug loaded film,
(e) layering the drug loaded film,
(f) fusing the layered film into a multilayer construct.

2. Process for the manufacturing of a multilayer construct according to claim 1 in which the layering (e) occurs via folding or rolling of one drug loaded film.

3. Process for the manufacturing of a multilayer construct according to claim 1 whereby at least two drug loaded films are layered.

4. Process for the manufacturing of a multilayer construct according to claim 3 whereby the drug loaded films may comprise the same or a different polymer and/or the same or a different drug.

5. Process for the manufacturing of a multilayer construct according to any one of the claims 1-4 in which the multilayer construct further comprises at least a non-drug loaded polymeric film.

6. Process for the manufacturing of a multilayer construct according to any one of the claims 1-4 in which the non-drug loaded film comprises the same or a different polymer as the drug loaded films.

7. Process for the manufacturing of a multilayer construct according to any one of the claims 1-6 wherein the overall thickness of the multilayer construct ranges from 500 nm - 2 cm.

8. Process for the manufacturing of a multilayer construct according to any one of the claims 1-7 whereby the drug is selected from the group of small molecule drugs or a biologic.

9. Process for the manufacturing of a multilayer construct according to claim 8 whereby the biologic is selected from the group of proteins, peptides or polynucleotides.

10. Process according to claim 1 whereby at least one of the polymeric sheets comprises an elastomeric material.

11. Process according to any one of the claims 1-8 whereby the polymer is a bioerodable or biodegradable polymer selected from the group consisting of polyesters or polyesteramides.

12. Process according to claim 11 whereby the polymer is selected from a polyesteramide comprising amino-acids.

13. Process according to claim 12 whereby the polyesteramide (PEA) has a chemical formula according to formula (I), 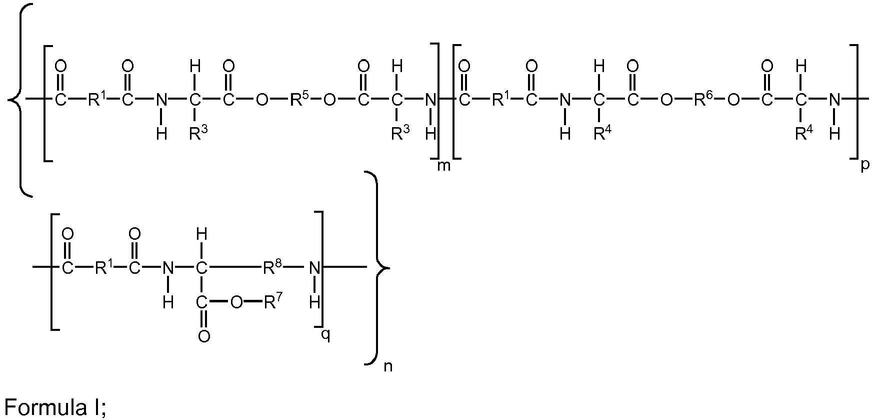 wherein
- m varies from 0.01 to 0.99; p varies from 0 to 0.99; and q varies from 0.99 to 0.01; and wherein n varies from 5 to 100; whereby
- R₁ is independently selected from the group consisting of (C₂-C₂₀) alkylene, (C₂-C₂₀) alkenylene and combinations thereof;
- R₃ and R₄ in a single backbone unit m or p, respectively, are independently selected from the group consisting of hydrogen, (C₁-C₆)alkyl, (C₂-C₆)alkenyl, (C₂-C₆)alkynyl, (C₆-C₁₀)aryl, (C₁-C₆)alkyl, -(CH₂)SH, - (CH₂)₂S(CH₃), -CH₂OH, -CH(OH)CH₃, -(CH₂)₄NH₃+, - (CH₂)₃NHC(=NH₂+)NH₂, -CH₂COOH, -(CH₂)COOH, -CH₂-CO-NH₂, - CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂-;
- R₅ is selected from the group consisting of (C₂-C₂₀)alkylene, (C₂-C₂₀)alkenylene, alkyloxy or oligoethyleneglycol;
- R₆ is selected from bicyclic-fragments of 1,4:3,6-dianhydrohexitols of structural formula (II); 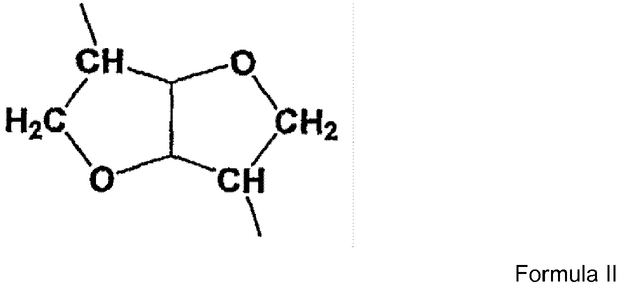
- R₇ is hydrogen, (C₆-C₁₀) aryl, (C₁-C₆) alkyl or a protecting group such as benzyl- or a bioactive agent;
- R₈ is -(CH₂)₄-.

14. Process according to claim 12 whereby the polyesteramide (PEA) has a chemical formula according to formula (III), 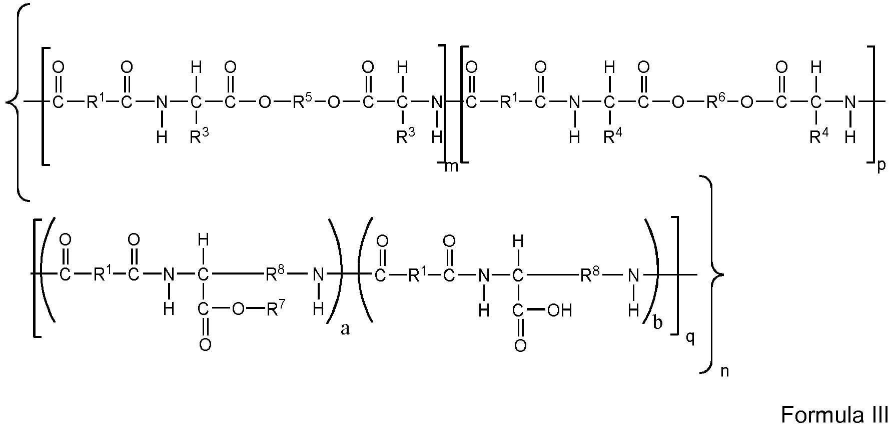 wherein
- m+p varies from 0.9-0.1 and q varies from 0.1 to 0.9 whereby m+p+q=1;
- m or p can be 0; a is at least 0.05, b is at least 0.05 whereby a+b=1
- n varies from 5 to 300;
- R₁ is independently selected from the group consisting of (C₂-C₂₀) alkylene, (C₂-C₂₀) alkenylene and combinations thereof;
-R₃ and R₄ in a single backbone unit m or p, respectively, are independently selected from the group consisting of hydrogen, (C₁-C₆)alkyl;
(C₂-C₆)alkenyl, (C₂-C₆)alkynyl, (C₆-C₁₀)aryl, (C₁-C₆)alkyl, -(CH₂)SH, - (CH₂)₂S(CH₃), -CH₂OH, -CH(OH)CH₃, -(CH₂)₄NH₃+, - (CH₂)₃NHC(=NH₂+)NH₂, -CH₂COOH, -(CH₂)COOH, -CH₂-CO-NH₂, - CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂-;
- R₅ is selected from the group consisting of (C₂-C₂₀)alkylene, (C₂-C₂₀)alkenylene, alkyloxy or oligoethyleneglycol;
- R₆ is selected from bicyclic-fragments of 1,4:3,6-dianhydrohexitols of structural formula II;
- R₇ is selected from the group consisting of (C₆-C₁₀) aryl (C₁-C₆) alkyl;
- R₈ is -(CH₂)₄-.

15. Process according to any one of the claims 1-14 in which the multilayer construct is further processed into a subsequent implantable or injectable drug delivery system such as a fiber, rod, disc, coating, tube or rolled film.

16. Process according to any one of the claims 1-15 wherein the multilayer construct or the implantable or injectable drug delivery system is used for the release of drugs in ophthalmology, cardiovascular, pain management, musculoskeletal, cancer treatment, in central nerve system or in vaccine delivery.

## Patentansprüche

1. Verfahren zum Herstellen eines mehrschichtigen Arzneimittelabgabekonstrukts, das wenigstens einen arzneimittelbeladenen Film umfasst, hergestellt durch die folgenden Schritte:
(a) Lösen des Polymers in einem Lösungsmittel,
(b) Mischen des gelösten Polymers mit einem Arzneimittel,
(c) Laminieren des Gemischs zwischen wenigstens zwei Polymerfolien, wobei wenigstens eine Polymerfolie für das Lösungsmittel durchlässig ist,
(d) Entfernen der Folien, um den arzneimittelbeladenen Film zu erhalten,
(e) Schichten des arzneimittelbeladenen Films,
(f) Verschmelzen des geschichteten Films zu einem mehrschichtigen Konstrukt.

2. Verfahren zum Herstellen eines mehrschichtigen Konstrukts gemäß Anspruch 1, wobei das Schichten (e) über Falten oder Walzen eines arzneimittelbeladenen Films erfolgt.

3. Verfahren zum Herstellen eines mehrschichtigen Konstrukts gemäß Anspruch 1, wobei wenigstens zwei arzneimittelbeladenen Filme geschichtet werden.

4. Verfahren zum Herstellen eines mehrschichtigen Konstrukts gemäß Anspruch 3, wobei die arzneimittelbeladenen Filme das gleiche oder verschiedene Polymere und/oder das gleiche oder verschiedene Arzneimittel umfassen können.

5. Verfahren zum Herstellen eines mehrschichtigen Konstrukts gemäß einem der Ansprüche 1-4, wobei das mehrschichtige Konstrukt ferner wenigstens einen nicht arzneimittelbeladenen Polymerfilm umfasst.

6. Verfahren zum Herstellen eines mehrschichtigen Konstrukts gemäß einem der Ansprüche 1-4, wobei der nicht arzneimittelbeladene Film das gleiche wie oder ein anderes Polymer als die arzneimittelbeladenen Filme umfasst.

7. Verfahren zum Herstellen eines mehrschichtigen Konstrukts gemäß einem der Ansprüche 1-6, wobei die Gesamtdicke des mehrschichtigen Konstrukts in dem Bereich von 500 nm bis 2 cm liegt.

8. Verfahren zum Herstellen eines mehrschichtigen Konstrukts gemäß einem der Ansprüche 1-7, wobei das Arzneimittel ausgewählt ist aus der Gruppe von kleinmolekulare Arzneimitteln und einem biologischen Stoff.

9. Verfahren zum Herstellen eines mehrschichtigen Konstrukts gemäß Anspruch 8, wobei der biologische Stoff ausgewählt ist aus der Gruppe von Proteinen, Peptiden und Polynukleotiden.

10. Verfahren gemäß Anspruch 1, wobei wenigstens eine der Polymerfolien ein elastomeres Material umfasst.

11. Verfahren gemäß einem der Ansprüche 1-8, wobei das Polymer ein bioerodierbares oder bioabbaubares Polymer ausgewählt aus der Gruppe bestehend aus Polyestern und Polyesteramiden ist.

12. Verfahren gemäß Anspruch 11, wobei das Polymer ausgewählt ist aus einem Polyesteramid, das Aminosäuren umfasst.

13. Verfahren gemäß Anspruch 12, wobei das Polyesteramid (PEA) eine chemische Formel gemäß Formel (I) aufweist, 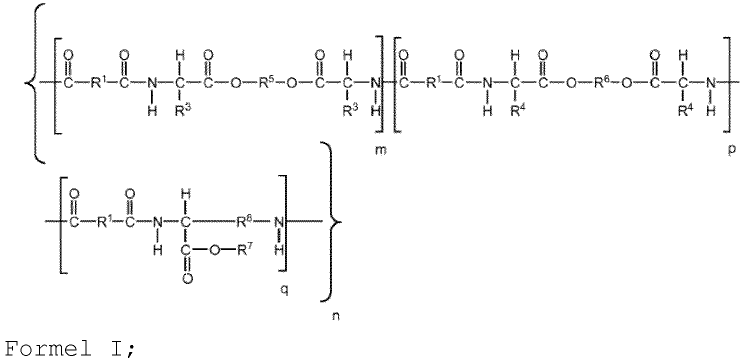 wobei
- m von 0,01 bis 0,99 variiert; p von 0 bis 0,99 variiert; und q von 0,99 bis 0,01 variiert; und wobei n von 5 bis 100 variiert; wobei
- R₁ unabhängig ausgewählt ist aus der Gruppe bestehend aus (C₂-C₂₀) Alkylen, (C₂-C₂₀) Alkenylen und Kombinationen davon;
- R₃ und R₄ in einer einzigen Gerüsteinheit m bzw. p unabhängig ausgewählt sind aus der Gruppe bestehend aus Wasserstoff, (C₁-C₆)Alkyl, (C₂-C₆) Alkenyl, (C₂-C₆)Alkinyl, (C₆-C₁₀) Aryl, (C₁-C₆)Alkyl, -(CH₂)SH, -(CH₂)₂S(CH₃), -CH₂OH, -CH(OH)CH₃, -(CH₂)₄NH₃⁺, -(CH₂)₃NHC(=NH₂⁺)NH₂, -CH₂COOH, -(CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂,
- R₅ ausgewählt ist aus der Gruppe bestehend aus (C₂-C₂₀) Alkylen, (C₂-C₂₀)Alkenylen, Alkyloxy und Oligoethylenglycol,
- R₆ ausgewählt ist aus bicyclischen Fragmenten von 1,4:3,6-Dianhydrohexitolen der Strukturformel (II); 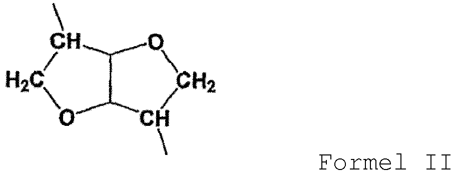
- R₇ Wasserstoff, (C₆-C₁₀) Aryl, (C₁-C₆)Alkyl oder eine Schutzgruppe, wie z. B. Benzyl-, oder ein bioaktives Mittel ist;
- R₈-(CH₂)₄ ist.

14. Verfahren gemäß Anspruch 12, wobei das Polyesteramid (PEA) eine chemische Formel gemäß Formel (III) aufweist, 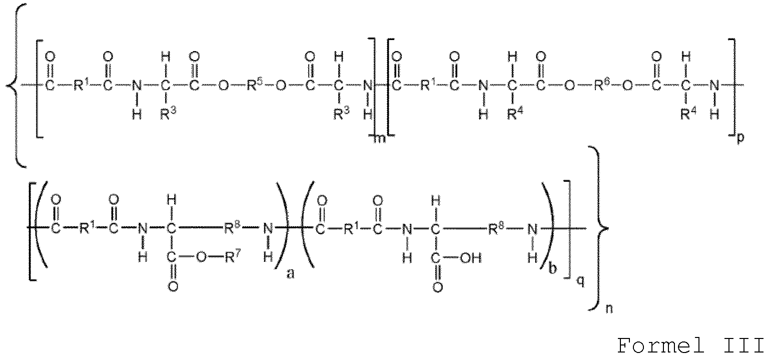 wobei
- m+p von 0,9 bis 0,1 variiert und q von 0,1 bis 0,9 variiert, wobei m+p+q = 1,
- m oder p 0 sein können; a wenigstens 0,05 ist, b wenigstens 0,05 ist, wobei a+b = 1,
- n von 5 bis 300 variiert,
- R₁ unabhängig ausgewählt ist aus der Gruppe bestehend aus (C₂-C₂₀) Alkylen, (C₂-C₂₀)Alkenylen und Kombinationen davon;
- R₃ und R₄ in einer einzigen Gerüsteinheit m bzw. p unabhängig ausgewählt sind aus der Gruppe bestehend aus Wasserstoff, (C₁-C₆)Alkyl, (C₂-C₆)Alkenyl, (C₂-C₆)Alkinyl, (C₆-C₁₀) Aryl, (C₁-C₆)Alkyl, -(CH₂)SH, -(CH₂)₂S(CH₃), -CH₂OH, -CH(OH)CH₃, -(CH₂)₄NH₃⁺, -(CH₂)₃NHC(=NH₂⁺)NH₂, -CH₂COOH, -(CH₂)COOH, -CH₂-CO-NH₂, -CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂,
- R₅ ausgewählt ist aus der Gruppe bestehend aus (C₂-C₂₀) Alkylen, (C₂-C₂₀)Alkenylen, Alkyloxy und Oligoethylenglycol,
- R₆ ausgewählt ist aus bicyclischen Fragmenten von 1,4:3,6-Dianhydrohexitolen der Strukturformel (II),
- R₇ ausgewählt ist aus der Gruppe bestehend aus (C₆-C₁₀)Aryl, (C₁-C₆)Alkyl,
- R₈-(CH₂)₄ ist.

15. Verfahren gemäß einem der Ansprüche 1-14, wobei das mehrschichtige Konstrukt ferner zu einem anschließend implantierbaren oder injizierbaren Arzneimittelabgabesystem verarbeitet wird, wie z. B. eine Faser, ein Stäbchen, eine Scheibe, eine Beschichtung, ein Rohr oder ein gerollter Film.

16. Verfahren gemäß einem der Ansprüche 1-15, wobei das mehrschichtige Konstrukt oder das implantierbare oder injizierbare Arzneimittelabgabesystem für die Freisetzung von Arzneimitteln in der Ophthalmologie, kardiovaskulär, bei dem Schmerzmanagement, muskuloskeletal, bei der Krebsbehandlung, in dem zentralen Nervensystem oder bei der Impfstoffabgabe verwendet wird.

## Revendications

1. Procédé de fabrication d'une structure multicouche d'administration de médicament comprenant au moins un film chargé de médicament fabriqué par les étapes suivantes :
(a) la dissolution du polymère dans un solvant ;
(b) le mélange du polymère dissous avec un médicament ;
(c) le laminage du mélange entre au moins deux feuilles polymères, au moins une feuille polymère étant perméable au solvant ;
(d) le retrait des feuilles pour produire le film chargé de médicament ;
(e) la stratification du film chargé de médicament ;
(f) l'assemblage par fusion du film stratifié pour produire une structure multicouche.

2. Procédé de fabrication d'une structure multicouche selon la revendication 1, dans lequel la stratification (e) a lieu par pliage ou enroulement d'un film chargé de médicament.

3. Procédé de fabrication d'une structure multicouche selon la revendication 1, dans lequel au moins deux films chargés de médicament sont stratifiés.

4. Procédé de fabrication d'une structure multicouche selon la revendication 3, dans lequel les films chargés de médicament peuvent comprendre le même polymère ou un différent polymère et/ou le même médicament ou un différent médicament.

5. Procédé de fabrication d'une structure multicouche selon l'une quelconque des revendications 1 à 4, dans lequel la structure multicouche comprend en outre au moins un film polymère non chargé de médicament.

6. Procédé de fabrication d'une structure multicouche selon l'une quelconque des revendications 1 à 4, dans lequel le film non chargé de médicament comprend le même polymère que les films chargés de médicament ou un différent polymère.

7. Procédé de fabrication d'une structure multicouche selon l'une quelconque des revendications 1 à 6, dans lequel l'épaisseur totale de la structure multicouche est de 500 nm à 2 cm.

8. Procédé de fabrication d'une structure multicouche selon l'une quelconque des revendications 1 à 7, dans lequel le médicament est sélectionné dans le groupe constitué de médicaments à petites molécules ou d'un agent biologique.

9. Procédé de fabrication d'une structure multicouche selon la revendication 8, dans lequel l'agent biologique est sélectionné dans le groupe constitué de protéines, de peptides ou de polynucléotides.

10. Procédé selon la revendication 1, dans lequel au moins l'une des feuilles polymères comprend un matériau élastomère.

11. Procédé selon l'une quelconque des revendications 1 à 8, dans lequel le polymère est un polymère bioérodable ou biodégradable sélectionné dans le groupe constitué de polyesters ou de polyesteramides.

12. Procédé selon la revendication 11, dans lequel le polymère sélectionné est un polyesteramide comprenant des acides aminés.

13. Procédé selon la revendication 12, dans lequel le polyesteramide (PEA) a une formule chimique selon la formule (I), 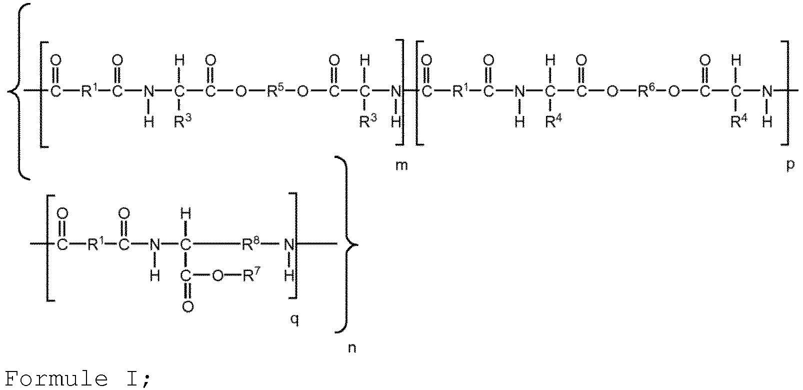 dans laquelle
- m vaut de 0,01 à 0,99 ; p vaut de 0 à 0,99 ; et q vaut de 0,99 à 0,01 ; et dans laquelle n vaut de 5 à 100 ;
- R₁ étant sélectionné indépendamment dans le groupe constitué d'un groupe alkylène C₂-C₂₀, d'un groupe alcénylène C₂-C₂₀ et de combinaisons de ceux-ci ;
- R₃ et R₄ dans une seule unité de squelette m ou p, respectivement, sont sélectionnés indépendamment dans le groupe constitué d'un atome d'hydrogène et de groupes alkyle C₁-C₆, alcényle C₂-C₆, alkynyle C₂-C₆, aryle C₆-C₁₀, alkyle C₁-C₆, -(CH₂)SH,-(CH₂)₂S(CH₃), -CH₂OH, -CH(OH)CH₃, -(CH₂)₄NH₃+,-(CH₂)₃NHC(=NH₂+)NH₂, -CH₂COOH, -(CH₂)COOH, -CH₂-CO-NH₂, - CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂- ;
- R₅ étant sélectionné dans le groupe constitué de groupes alkylène C₂-C₂₀, alcénylène C₂-C₂₀, alkyloxy ou oligoéthylène glycol ;
- R₆ étant sélectionné parmi des fragments bicycliques de 1,4:3,6-dianhydrohexitols ayant la formule structurale (II); 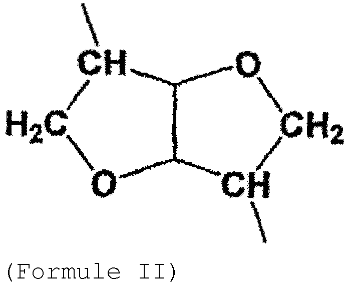
- R₇ étant un atome d'hydrogène, un groupe aryle C₆-C₁₀, un groupe alkyle C₁-C₆, ou un groupe protecteur tel qu'un groupe benzyl- ou un agent bioactif ;
- R₈ étant un groupe -(CH₂)₄-.

14. Procédé selon la revendication 12, dans lequel le polyesteramide (PEA) a une formule chimique selon la formule (III), 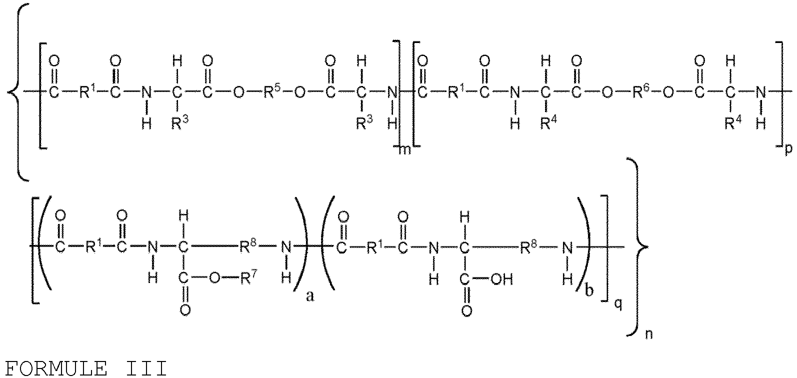 dans laquelle
- m + p vaut de 0,9 à 0,1 et q vaut de 0,1 à 0, 9, et m + p + q = 1 ;
- m ou p peut valoir 0 ; a vaut au moins 0,05, b vaut au moins 0,05, et a + b = 1 ;
- n vaut de 5 à 300 ;
- R₁ étant sélectionné indépendamment dans le groupe constitué d'un groupe alkylène C₂-C₂₀, d'un groupe alcénylène C₂-C₂₀ et de combinaisons de ceux-ci ;
- R₃ et R₄ dans une seule unité de squelette m ou p, respectivement, sont sélectionnés indépendamment dans le groupe constitué d'un atome d'hydrogène et de groupes alkyle C₁-C₆, alcényle C₂-C₆, alkynyle C₂-C₆, aryle C₆-C₁₀, alkyle C₁-C₆, -(CH₂)SH,-(CH₂)₂S(CH₃), -CH₂OH, -CH(OH)CH₃, -(CH₂)₄NH₃+,-(CH₂)₃NHC(=NH₂+)NH₂, -CH₂COOH, -(CH₂)COOH, -CH₂-CO-NH₂, - CH₂CH₂-CO-NH₂, -CH₂CH₂COOH, CH₃-CH₂-CH(CH₃)-, (CH₃)₂-CH-CH₂-, H₂N-(CH₂)₄-, Ph-CH₂-, CH=C-CH₂-, HO-p-Ph-CH₂-, (CH₃)₂-CH-, Ph-NH-, NH-(CH₂)₃-C-, NH-CH=N-CH=C-CH₂- ;
- R₅ étant sélectionné dans le groupe constitué de groupes alkylène C₂-C₂₀, alcénylène C₂-C₂₀, alkyloxy ou oligoéthylène glycol ;
- R₆ étant sélectionné parmi des fragments bicycliques de 1,4:3,6-dianhydrohexitols ayant la formule structurale II ;
- R₇ étant sélectionné dans le groupe constitué d'un groupe aryle C₆-C₁₀ et d'un groupe alkyle C₁-C₆ ;
- R₈ étant un groupe -(CH₂)₄-.

15. Procédé selon l'une quelconque des revendications 1 à 14, dans lequel la structure multicouche est en outre transformée en un système d'administration de médicament implantable ou injectable subséquent, tel qu'une fibre, une tige, un disque, un revêtement, un tube ou un film enroulé.

16. Procédé selon l'une quelconque des revendications 1 à 15, dans lequel la structure multicouche ou le système d'administration de médicament implantable ou injectable est utilisé(e) pour la libération de médicaments en ophtalmologie, en médecine cardiovasculaire, pour la gestion de la douleur, dans le système musculo-squelettique, pour le traitement du cancer, dans le système nerveux central ou dans l'administration de vaccins.
